# EUROPEAN PATENT SPECIFICATION

(11) **EP 3 650 048 B1**
(45) Date of publication and mention of the grant of the patent: **05.03.2025**
(21) Application number: 19159230.2
(22) Date of filing: 26.09.2013
(51) Int. Cl.: A61K 49/00, A61K 9/16, A61K 9/20, A61K 9/00, A61K 9/28

(54) **SOLID ORAL COMPOSITION CONTAINING DYES FOR USE IN ENDOSCOPIC DIAGNOSIS**
ORALE FESTSTOFFZUSAMMENSETZUNG MIT FARBSTOFFEN FÜR DIE VERWENDUNG BEI DER ENDOSKOPISCHEN DIAGNOSE
COMPOSITION ORALE SOLIDE CONTENANT DES COLORANTS CONVENANT POUR DIAGNOSTIC ENDOSCOPIQUE

(30) Priority: 19.10.2012 EP 12189206; 19.10.2012 US 201261715981 P
(43) Date of publication of application: 13.05.2020
(62) Divisional of application: 13774382.9
(73) Proprietor: Cosmo Technologies Ltd., Dublin 2 (IE)
(72) Inventor: MORO, Luigi, 21050 Cairate (IT); REPICI, Alessandro, 10123 Torino (IT)
(74) Representative: FRKelly

(56) References cited:
- WO-A1-2011/107945
- US-A1- 2007 077 202
- MITOOKA ET AL: "Minute flat depressed neoplastic lesions of the colon detected by contrast chromoscopy using an indigo carmine capsule", GASTROINTESTINAL ENDOSCOPY, ELSEVIER, NL LNKD- DOI:10.1016/S0016-5107(05)80003-3, vol. 41, no. 5, 1 May 1995 (1995-05-01), pages 453 - 459, XP005452014, ISSN: 0016-5107
- PARRA-BLANCO ET AL.: "colonoscopy with an indigo carmine capsule: a controlled trial", GASTROENTEROLOGY, vol. 130, 22 July 2006 (2006-07-22), pages A187, XP002599122

## Description

### DESCRIPTION

Endoscopy is an exceptionally important diagnostic technique for the diagnosis of inflammatory, ulcerative, and neoplastic pathologies of the gastrointestinal tract.

Actually, endoscopy allows observing - from inside the lumen - the state of preservation and development of the mucosa that covers the gastrointestinal cavity, as well as the surface spraying thereof, the presence of deformations, and/or neoformations, and/or ulcerations.

Increasingly more powerful and sophisticated endoscope probes have considerably improved this technique. The progress of the materials employed has also improved performance in terms of illumination technologies and resolution power.

More recently, there has been an improvement of the conventional diagnostic therapeutic aspects involving image magnification and vital dyes, used to locally develop a contrasting colour capable of amplifying the resolution diagnostic power of the conventional technique. The use of dyes in diagnostic endoscopic procedures is described by "chromoendoscopy", particularly useful for identifying suspicious areas displaying degenerative characteristics.

The use of colouring is generally adopted in the second part of the endoscopic analysis, during the step of withdrawing the endoscopic probe, and after accurately cleaning the mucosa tract to be examined. Currently, the dye is applied to the mucosa by spraying a certain volume of a dye-containing solution using a catheter or capillary pipe directly inserted into the working channel of the endoscopic probe.

The diffusion of the dye on the cell surface or the extent of absorption by the vital cells markedly differentiates the cells with normal vitality from those cells, such as neoplastice cells, in the advanced replication stage.

The dyes usually used are mainly, but not exclusively, the following: methylene blue, congo red, carmine indigo, and/or toluidine blue.

Methylene blue and toluidine blue are uniformly absorbed by the whole intestinal mucosa but that absorption is reduced in an inflammatory environment, particularly as the phlogosis, i.e, inflammation, worsens. Due to this characteristic, the two dyes are also useful to ascertain whether inflammatory processes are in remission, and are also useful in distinguishing between pseudopolyps and true polyps. Indeed, inflamed or malignant/premalignant colonic epithelium exhibits decreased cytoplasm and goblet cells that are either reduced in amount or absent. These alterations result in decreased uptake of methylene blue and endoscopic appearance of focal light blue or pink (unstained) or heterogeneously stained (specked) mucosa in contrast to a more uniform staining pattern when colonic mucosa is not affected by pathologic processes. Differently from this concept, carmine indigo is not absorbed by cells and functions as a contrast agent increasing visibility of mucosal structures and enhancing details of normal and abnormal colonic patterns. Carmine *indigo* thus finds application in long duration inflammatory forms and can be used to highlight flat lesions, which can contain tumoral forms, which are difficult to detect with conventional white light endoscopy that does not employ contrasting colours.

Within the dyeing procedure, it should be observed that use thereof reveals several practical problems that can be difficult to resolve due to the challenges involved in applying the dye. First and foremost the pharmacy of the institute where the endoscopy is performed should be capable of preparing solutions with concentrations of dye generally ranging from 0.1% to 1%; then the dye should be dispensed (using a dedicated spray catheter) uniformly so as to cover homogeneously the mucosal surface subject of the evaluation.

Furthermore, the sprayed dye excess is to be removed after a few minutes through washing and sucking operations. That removal of excess dye requires additional time after each repetition of the dyeing spray process during the colonoscopy. The process, consequently, is time consuming for both nurses and physicians and makes it difficult to maximize the efficiency of the schedule of endoscopic procedures. The procedure is sufficiently rare that it tends to be operator-dependent, requiring a dedicated learning curve to obtain the right level of expertise to be able to evaluate the specific staining patterns obtained and their significance.

The need for the simultaneous presence of these precise conditions contributes to the difficulty of executing the chromoendoscopy procedure. Those difficulties have resulted in the procedure being carried out by only a minority of endoscopy units in hospitals and nursing homes specialized in gastroenterology.

Furthermore, other problems have resulted. The conventional local spraying of a solution on the mucosal wall may fail to reveal forms that are latent but still too small to detect and may fail to reveal the degenerative processes of the digestive system.

Moreover, locally spraying a solution can result in a short performance time of the dye. In particular, the time between spraying of the dye and observation is generally only a few seconds or a couple of minutes, a period known to be too short for allowing a consistent absorption of the dye to provide good contrast development and also achievement of good staining efficacy. Those issues may make it difficult for the endoscopist to intervene to obtain good detection and evaluation, as for example, in a biopsy.

Furthermore, the experience of each endoscopist who performs the procedure is somewhat subjective, additionally generating problems in the execution of both the endoscopic and related diagnostic evaluations. As a practical difficulty, such subjectivity resulting from the experience and convenience of the operator can undesirably lead to great variability in results. And the experience of the endoscopist plays an important role: the more experienced endoscopist, compared to the less experienced endoscopist, may spot suspicious areas when the dye is sprayed according to the current chromoendoscopy, further exacerbating the subjectivity of the test results.

Significant variability in test results can also result from the apparatus used, as well as from the acceptability of a particular patient to the diagnostic evaluation practice.

Thus, there arises the need of providing further improvement in both simplicity and safety from use of a dye in diagnostic endoscopies. It is desirable to improve the means of administration to provide a homogeneous and complete distribution of the dye for an improved effect in evaluating a treated area.

WO2011107945A1 describes solid compositions for the oral administration of dyes, and diagnostic use thereof. Preferably, such diagnostic use is aimed at the diagnostic evaluation of the gastrointestinal tract.

Mitooka et al. (Gastrointset. Endosc. 1992, 1995, 41, 453-459) describes the detection of minute flat depressed neoplastic lesions of the colon by contrast chromoscopy using an indigo carmine capsule and the subsequent removal of said lesions by endoscopic mucosal resection.

Parra-Blanco et al. Gastroenterology, vol. 130, 2006, page A187 describes a randomized controlled trial wherein colonoscopy was carried out with an indigo carmine capsule.

US2007077202A1 describes a histostain composition for endoscope containing one or more members selected from colors derived from Monascus. The stain composition is a staining agent which sharpens the shapes of digestive tract lumen surfaces and the like with a light in the visible wavelength range, having a function of being excited by a light of specific wavelength to emit fluorescence, and being biologically safe and suitable for endoscopy.

And as will be evident from above, it is desirable to obtain improvements that will increase the objectivity of the endoscopic evaluation to allow an improved diagnostic evaluation. Particularly, in the case of colonic endoscopy (colonoscopy), a need still exists for providing an improved mucosal staining and ameliorating the efficacy of the diagnostic endoscopy evaluation.

It has been surprisingly discovered that a specific solid composition containing at least one dye and at least one physiologically acceptable excipient, orally administered according to a defined fractionated schedule prior to endoscopy, can provide an improved mucosal staining. And, increasing the objectivity of the endoscopy, the solid composition disclosed herein can also provide an improved detection characterization in endoscopic diagnosis.

In one aspect the present invention provides a solid composition for use in endoscopic evaluation as set out in the claims.

Disclosed herein is a solid composition containing at least one dye in association with at least one physiologically acceptable excipients which comprises:
a) a matrix which comprises lipophilic compounds with melting point below 90°C, and optionally amphiphilic compounds, in which matrix at least one dye is at least partly incorporated,
b) an outer matrix which comprises hydrophilic compounds, in which the lipophilic matrix, and optionally the amphiphilic matrix are dispersed;
   a) optionally other physiologically acceptable excipients;
   b) optionally a gastro-resistant coating
      for use in endoscopic diagnosis characterised in that more than two unit dosages of the solid composition are orally administered to a human according to a fractionated schedule in which a total amount from 100 to 400 mg of said at least one dye is administered to a human in the 48 hours prior to endoscopic diagnosis. For example, said at least one dye is administered to a human in the 24 hours prior to endoscopic diagnosis.

In the alternative, the matrix consists of lipophilic compounds with melting point below 90°C, and optionally amphiphilic compounds, in which matrix at least one dye is at least partly incorporated, and the outer matrix consists of hydrophilic compounds, in which the lipophilic matrix, and optionally the amphiphilic matrix are dispersed.

Said more than two unit dosages are, for example, four, six or eight unit dosages administered in the 48 hours prior to endoscopy, such as in the 24 hours prior to endoscopy.

Useful dyes according to the present disclosure can be selected from among congo red, carmine indigo, methylene blue, toluidine blue or mixtures thereof; for example, the dye may be methylene blue.

According to the disclosure herein, methylene blue can be in anhydrous or hydrated forms, such as the trihydrate form.

However, according to the disclosure other biocompatible dye substances can also be used, as long as they are provided with a toxicity profile that does not represent an obstacle to oral systemic administration thereof.

A "fractionated schedule" according to the disclosure means that the total amount of the dye to be orally administered before colonoscopy is divided in more than two unit dosages to obtain a pre-defined administration schedule. The dose fractionation can reduce the possibility that staining will be lost due to unwanted strange intestinal motility. And the dose fractionation can facilitate the spreading of the blue staining matrices.

The endoscopic diagnosis as disclosed herein is directed to the gastro-intestinal tract, such as the colon (colon endoscopy or colonoscopy). According to the anatomical classification, the colon is divided into four (4) regions of interest (ROI), namely (1) ascending colon (AC), (2) transverse colon (TC), (3) descending colon (DC), and (4) rectosigmoid (RES).

As disclosed herein, the total dose amount of said at least one dye is, for example, from 100 to 400 mg, such as from 100 to 250 mg, and further such as 200 mg.

As disclosed herein, the unit dosage of the composition contains, for example, from 20 to 200 mg by weight of the at least one dye. For example, said unit dosage contains about 25 mg or about 50 mg, such as 25 mg or 50 mg, by weight of said at least one dye. According to an embodiment disclosed herein, eight unit dosages of the composition, each containing about 25 mg, such as 25 mg, by weight of said at least one dye, are administered to said human in the 48 hour period prior to endoscopic diagnosis. According to another embodiment disclosed herein, six unit dosages of the composition, each containing about 25 mg, such as 25 mg, by weight of said at least one dye, are administered to said human in the 48 hour period prior to endoscopic diagnosis. According to a yet one other embodiment disclosed herein, four unit dosages of the composition of the invention, each containing about 25 mg, such as 25 mg, by weight of said at least one dye, are administered to said human in the 48 hour period prior to endoscopic diagnosis.

According to a further embodiment disclosed herein, four unit dosages of the composition, each containing about 50 mg, such as 50 mg, by weight of said at least one dye, are administered to said human in the 48 hour period prior to endoscopic diagnosis.

As disclosed herein, to facilitate the mucosal observation through the endoscope by the endoscopist, said human, prior to endoscopic diagnosis, can be subjected to a bowel cleansing preparation by the administration of bowel cleansing solution to quantitatively remove the stool and mucous residuals. This cleansing operation is carried out generally in the 48 hour period prior to endoscopic diagnosis, such as in the 24 hour period prior to endoscopic diagnosis or, as found to be practical for carrying out a colonoscopy in the late afternoon, also in the same day.

The colon cleansing preparation could be administered by drinking the volume fractions of the cleansing solution consecutively during the day before or, with the so called "split" version, by dividing the administration of the cleansing solution volume in two parts, one to be administered the day before the colonoscopy and one to be administered in the morning of the day in which the colonoscopy is to be subsequently performed.

The bowel cleansing solution is used for cleaning and washing the intestinal tract and mucosa before the endoscopic diagnosis. The bowel cleansing solution is, for example, a saline and/or polyethylenglycol (PEG) aqueous solution, such as a polyethylene glycol aqueous solution. As a further example, said aqueous solution contains, excluding water, from 50%.to 95% by weight of polyethylene glycol, sometimes also including into that solution, salts and flavours, such as sodium salts, potassium salts, ascorbic acid, and mixtures thereof. For example, sodium sulphate, sodium sulphate anhydrous, sodium chloride, sodium ascorbate, sodium bicarbonate, sodium salt of ascorbic acid, potassium sulphate, potassium chloride and mixtures thereof can be used. As a further example, the bowel cleansing solution is an aqueous solution of commercially available products sold under such names as Moviprep^{®} or Golytely^{®}, Nulytely^{®}, or Halflytely^{®}, or Movicol^{®}, or Macro-P^{®}, or Colirei^{®}, or Isocolan^{®} or Selg 1000^{®}.

However, as disclosed herein, also other bowel cleansing solutions or preparations can be used, as long as they are provided with a toxicity profile that does not represent an obstacle to oral systemic administration thereof. For example, bowel cleansing solution containing only salts or other small chemical laxatives, but not PEG, are available on the market under the brands Phospho-Lax^{®} or Picoprep^{®} or Suprep^{®}. Also different bowel preparation procedures can be used.

As disclosed herein, the cleansing solution can be administered in a total amount of four litres, which can be fractionated in one or more unit dosages, for example, in four unit dosages of about one litre each.

The solid composition, as disclosed herein, can be thus administered together and/or after the intake of each unit dosage of said bowel cleansing solution, prior to the endoscopic diagnosis. Afterwards, still water can also be additionally administered, if necessary.

As disclosed herein, four unit dosages of the composition, each containing about 25 mg, such as 25 mg, by weight of said at least one dye, are orally administered to a human according to a fractionated schedule in which a total amount of about 100 mg, such as 100 mg, of said at least one dye is administered to said human in the 48 hour period prior to the endoscopic diagnosis in:
- 1 solid oral composition after intake of the 1st litre of bowel cleansing solution;
- 1 solid oral composition after intake of the 2nd litre of bowel cleansing solution;
- 1 solid oral composition after intake of the 3nd litre of bowel cleansing solution;
   and
- 1 solid oral composition after intake of the 4th (and last) litre of bowel cleansing solution.

As disclosed herein, eight unit dosages of the composition, each containing about 25 mg, such as 25 mg, by weight of said at least one dye, are orally administered to a human according to a fractionated schedule in which a total amount of about 200 mg, such as 200 mg, of said at least one dye is administered to said human in the 48 hour period prior to the endoscopic diagnosis in:
- 2 solid oral compositions after intake of the 1st litre of bowel cleansing solution;
- 2 solid oral compositions after intake of the 2nd litre of bowel cleansing solution;
- 2 solid oral compositions after intake of the 3rd litre of bowel cleansing solution;
   and
- 2 solid oral compositions after intake of the 4th (and last) litre of bowel cleansing solution.

For example, eight unit dosages of the composition as disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 48 hour period prior to the endoscopic diagnosis in:
- 0 solid oral compositions after intake of the 1st litre of bowel cleansing solution;
- 2 solid oral compositions after intake of the 2nd litre of bowel cleansing solution
- 3 solid oral compositions after intake of the 3rd litre of bowel cleansing solution;
   and
- 3 solid oral compositions after intake of the 4th (and last) litre of bowel cleansing solution.

As a further example, eight unit dosages of the composition disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 48 hour period prior to the endoscopic diagnosis in:
0 solid oral compositions after intake of the 1st litre of bowel cleansing solution;
4 solid oral compositions after intake of the 2nd litre of bowel cleansing solution;
4 solid oral compositions after intake of the 3nd litre of bowel cleansing solution; and
0 solid oral compositions after intake of the 4th litre of bowel cleansing solution.

As a yet further example, eight unit dosages of the composition as disclosed herein, each containing about 25 mg, such as 25 mg, by weight of said at least one dye, are orally administered to a human according to a fractionated schedule in which a total amount of about 200mg, such as 200mg, of said at least one dye is administered to said human in the 48 hour period prior to the endoscopic diagnosis in:
- 0 solid oral compositions after intake of the 1st litre of bowel cleansing solution;
- 3 solid oral compositions after intake of the 2nd litre of bowel cleansing solution;
- 3 solid oral compositions after intake of the 3nd litre of bowel cleansing solution;
   and
- 2 solid oral compositions after intake of the 4th litre of bowel cleansing solution.

As further disclosed within, four unit dosages of the composition as disclosed herein, each containing about 25 mg, such as 25 mg, by weight of said at least one dye, are orally administered to a human according to a fractionated schedule in which a total amount of about 100mg, such as 100mg, of said at least one dye is administered to said human in the 48 hour period prior to the endoscopic diagnosis in:
- 0 solid oral composition after intake of the 1^{st} litre of bowel cleansing solution;
- 1 solid oral composition after intake of the 2^{nd} litre of bowel cleansing solution;
- 1 solid oral compositions after intake of the 3^{rd} litre of bowel cleansing solution;
   and
- 2 solid oral compositions after intake of the 4^{th} (and last) litre of bowel cleansing solution.

As even further disclosed herein, six unit dosages of the composition, each containing about 25 mg, such as 25 mg, by weight of said at least one dye, are orally administered to a human according to a fractionated schedule in which a total amount of about 150 mg, such as 150 mg, of said at least one dye is administered to said human in the 48 hour period prior to the endoscopic diagnosis in:
- 2 solid oral composition at the begriming of bowel preparation, before intake of the 1st litre of bowel cleansing solution;
- 2 solid oral compositions after intake of the 1^{st} litre of bowel cleansing solution;
- 2 solid oral compositions after intake of the 2^{nd} litre of bowel cleansing solution
- 0 solid oral compositions after intake of the 3^{rd} litre of bowel cleansing solution;
   and
- 0 solid oral compositions after intake of the 4^{th} (and last) litre of bowel cleansing solution.

As yet another further example, the above indicated administration schedule can be carried out applying also the "split" bowel cleansing procedure. In such a case, the tablet administration is split over the two days of bowel cleansing preparation, maintaining the relevant schedule here described. Examples of the split preparation, according to further example disclosed herein, are here below detailed:
eight unit dosages of the composition disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 24 hour period prior to the endoscopic diagnosis in a split preparation procedure, where:
- 3 solid oral compositions after intake of the 1^{st} litre of bowel cleansing solution the day before colonoscopy;
- 3 solid oral compositions after intake of the 2^{nd} litre of bowel cleansing solution the day before colonoscopy;
- 2 solid oral compositions after intake of the 3^{rd} litre of bowel cleansing solution the same day of colonoscopy; and
- 0 solid oral compositions after intake of the 4^{th} litre of bowel cleansing solution the same day of colonoscopy.

Alternatively, as a further example, eight unit dosages of the composition disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 24 hour period prior to the endoscopic diagnosis in a split preparation procedure, where:
- 0 solid oral compositions after intake of the 1^{st} litre of bowel cleansing solution the day before colonoscopy;
- 6 solid oral compositions during the intake of the 2^{nd} litre of bowel cleansing solution the day before colonoscopy;
- 2 solid oral compositions after intake of the 3^{rd} litre of bowel cleansing solution the same day of colonoscopy; and
- 0 solid oral compositions after intake of the 4^{th} litre of bowel cleansing solution the same day of colonoscopy.

The solid composition disclosed herein can be a controlled release composition. The expression "controlled release" of the composition disclosed herein is used to indicate a composition capable of releasing the dye in a selective site-time manner, i.e. progressive in the areas of interest. Thus, such expression comprises the "prolonged, sustained, extended, delayed or modified" release definition.

The technology suitable for the formulation of controlled release composition disclosed herein can be selected from the colonic specific release technologies, utilized with matrix structures, and the reservoir structure as systems, using dissolution controlling mechanisms and technologies known in the art, such as diffusion, swelling, and macromolecular relaxation.

The oral composition disclosed herein can be formulated according to the multimatrix technology commercially known under the trade mark MMX^{®}, described in the international patent applications WO2011/107945, WO00/76481 and WO00/76478 and copending U.S. patent application Serial No. 13/602,875 filed on 4 September 2012, the disclosures of which are relevant to multimatrix technology

Suitable lipophilic compounds as disclosed herein can be selected from saturated, unsaturated and hydrogenated long chain alcohols, saturated and unsaturated and hydrogenated fatty acids, salts thereof, esters and amides, mono-, di- and triglycerides of fatty acids, polyethoxylated derivatives thereof, waxes, ceramides, cholesterol, cholesterol derivatives and mixtures thereof having a melting point lower than 90°C, such as from 40 to 90°C, and further such as from 60 to 70°C.

Suitable amphiphilic compounds as disclosed herein can be selected from among polar lipids of type I and II (lecithin, phosphatidylcholine, phosphatidylethanolamine, and mixtures thereof), ceramides, glycol alkyl ethers (such as for example, diethylene glycol monomethyl ether), alkyl sulfate and sulfosuccinate salts, and mixtures thereof.

Suitable hydrophilic compounds as disclosed herein can be chosen from compounds forming hydrogel (i.e. compounds which form hydrogel on contact with aqueous solvents), such as those selected from among polymers and copolymers of acrylic acid, copolymers of methacrylic acid, alkyl vinylpolymers, alkyl celluloses, hydroxyalkyl celluloses, carboxyalkyl cellulose, modified and/or plurisubstituted celluloses, polysaccharides, dextrins, pectins, starches, complex starches and starch derivatives, alginic acid, synthetic rubber, natural rubber, polyalcohols and mixtures thereof.

Hydrogels are compounds which when passing from the dry state to the hydrated one undergo so-called "molecular relaxation", namely a remarkable increase in mass and weight following the coordination of a large number of water molecules by the polar end groups present in the polymeric chains of the excipients themselves.

A suitable gastro-resistant coating, as disclosed herein, can be chosen from polymers of acrylic acid, polymers of methacrylic acid, copolymers of acrylic acid, copolymers of methacrylic acid, cellulose derivatives (such as for example cellulose acetate phthalate) hydroxybutyrate-based polymers, shellac and mixtures thereof. Such gastro-resistant coatings of the invention can also be combined with plasticisers, opacifiers, dyes and mixtures thereof.

The administration of a controlled release composition as disclosed herein actually allows releasing the dye contained in the composition precisely starting from the gastrointestinal segment intended to be subjected to endoscopic evaluation, such as in the intestinal regions and even further such as in the colonic regions.

The composition as disclosed herein is formulated in forms chosen from tablets, capsules, granules, microgranules, and pellets, such as in the form of a coated tablet, further such as in the form of gastro-protected tablets.

The capsule form disclosed herein may in turn contain granules, microgranules and/or pellets.

For example, the composition described herein may be formulated in the form of gastro-resistant tablets or in the form of a capsule containing gastro-resistant granules, gastro-resistant microgranules and/or gastro-resistant pellets.

Furthermore, the composition disclosed herein may be formulated in a double layer form, such as a double layer tablet.

As disclosed herein, in case of colonoscopy, more than two unit dosages of the compositions disclosed herein may be provided for the oral administration of more than two unit dosages of the compositions described herein, such as a controlled release tablet, so as to prevent the dye from being dispersed into areas of the digestive tract not intended to be subjected to colonoscopy, such as, for example, the stomach, duodenum and jejunum.

For the preparation of controlled release compositions, one or more dyes can be formulated alongside substances capable of imparting progressive or massive or controlled or prolonged dissolution properties to the formulation. In addition, the formulation is coated with substances capable of dissolving solely upon reaching a specific pH, generally running from pH 5 to pH 7, that pH being typical of the section intended to be subject to the intestinal endoscopic evaluation.

Upon reaching the intestinal section of interest, characterised by a specific pH value at which the gastro-protective coating starts dissolving, the dissolution of the dye can be controlled in terms of speed so as to ensure that it occurs within the time required by the intestinal transit, such as the time to reach the colon, generally running from 4 to 24 hours. As disclosed herein, the dye/s is/are first mixed or granulated with the material capable of forming a lipophilic matrix, such as in the presence of one or more amphiphilic substances with surfactant properties, and lastly this matrix of powders, at any degree of aggregation, is inserted into a dominant structure formed by polymers or copolymers of the hydrophilic type, also known as hydrogels, in the anhydrous state or with some residual moisture value.

Alternatively, still according to a typical application of this technology, the dye/s should be first mixed or granulated with the material capable of forming a lipophilic matrix, and after granulation this matrix structure, at any degree of aggregation, is inserted into a dominant structure formed by polymers or copolymers of hydrophilic type in anhydrous state or with some residual moisture value in the presence, for example, of one or more amphiphilic substances with surfactant properties. Subsequently the final mixture is subjected to compression.

A gastro-protective coating film, capable of preventing the dissolution of the composition in a strongly acid environment, can be lastly applied to the surface of the compositions.

Upon swallowing; such a multimatrix coated composition can be protected from contact with gastric and intestinal acids up to reaching an environment with suitable pH, such as greater than 5 or 7, where the gastro-protective coating is solubilised and where the dissolution program - which will lead it to progressively distribute the dye inserted in the formulation simultaneously with the progress of transit within the digestive cavity - starts. The endoscopic diagnosis disclosed herein is aimed at the diagnosis of inflammatory, ulcerative, pre-neoplastic, dysplastic and/or neoplastic pathologies and/or alterations of the gastrointestinal tract, such as of the colon and further such as the right part of the colon.

For example, the endoscopic diagnostic evaluation disclosed herein can be aimed at the diagnosis of cancerous forms, precancerous forms, interval cancers, adenomas, carcinomas, serrated lesions, dysplasias, polyps, pseudopolyps, pre-polyps hyperplastic lesions and different inflammatory pathologies and/or lesions of the gastrointestinal tract, such as of the colon and further such as of the right part of the colon.

The endoscopic diagnosis of the right part of the colon can also be aimed at the diagnosis of right colon adenomas, right colon polyps, serrated adenomas and right serrated lesions or interval cancers.

An interval cancer relates to lesions able to become cancers (tumours) in the time between two consecutive colon endoscopies (colonoscopies). Such time generally corresponds to a period of 2-5 years.

The oral composition disclosed herein can be aimed to increase and to improve the diagnosis of those small size lesions and flat lesions that are mostly missed during white light colonoscopy. As used herein, the term "small size" is a size equal to or less than 10 mm, such as equal or less than 5 mm. For example, polyps, adenomas and serrated lesion of the right colon of size less than 5 mm in diameter are considered to be "small size." The size is determined as the diameter of lesion estimated or measured by using a standard foreign body forceps.

These right colon lesions are in fact considered difficult to be seen and detected in this field, because of the anatomical conformation of the colon mucosal tissues and the possibility to have an unclean mucosal surface, that would make the lesion's detection very difficult in standard white light colonoscopy practice.

Also, the smaller colon lesions are the more difficult to be selected because of the possibility to be confused with the colonic plicas, as well as the possibility of having an unclean mucosal surface that hides such smaller lesions, thus making those smaller lesions difficult to detect.

As disclosed herein, the endoscopic diagnosis can also be aimed at the diagnosis of the above mentioned pathologies and/or lesions in a human previously suffering from at least another inflammatory pathology as, for example, Inflammatory Bowel Disease (IBD), Ulcerative Colitis or Crohn's Disease.

In that case, said human is indicated to be a "more risky patient". In this kind of patients, in fact, the risk of subsequent pathologies and/or lesions of the intestinal and colonic mucosa is much higher than normal because the mucosa is affected by chronic flogistic processes that in the long-term may be associated with uncontrolled cell proliferation and neoplastic development. Particularly, the risk significantly increases at colonic level where for example colon carcinoma and/or colon dysplasia and/or intraepithelial neoplasias can more likely arise in patients with long-standing ulcerative colitis and Crohn' s disease.

A first advantage of the oral composition disclosed herein is to provide an improved staining quality and staining efficacy in the area to be investigated by the endoscopic diagnostic evaluation, such as the colon regions (ascending, descending, rectosigmoid and transverse colon) and even further such as the right part of the colon.

This improved staining quality is related to a number of different factors. First, the dye is quite homogenously delivered throughout the entire length of the bowel according to the multi-matrix delivery system and the specific schedule of dye administration which ensures long-lasting and anatomically consistent availability of the coloring substance. Second and foremost, the disclosure herein allows for the first time a certain interval time between the dye contact with the colonic mucosa and the endoscopic procedure.

This interval time is relevant, allowing for proper dye absorption in the mucosa which becomes consistently coloured thanks to the incorporation of the blue substance into the cells. Selective dye absorption is considered the pivotal mechanism of action of vital dyes like methylene blue.

Indeed this absorption and the consequent enhanced contrast is minimally obtained when the dye is sprayed during the endoscopic procedures. The absorption is maximized when a certain interval lasts between dye delivery and endoscopic procedure.

The third factor leading to an improved staining is strictly related to colonic anatomy. Indeed the right colon has a larger lumen and a greater mucosal surface as compared to other colonic segments.

According to these facts and because of a gravity issue (during the endoscopic procedure the patients lay down in a supine position) when the dye is sprayed at the time of endoscopic procedure, the dye tends to distribute in a patchy mode, for example, in the most downward part of the mucosa (because of gravity).

Differently from this situation, in the condition of a targeted oral delivery of the dye with an MMX mechanism, at least 5 hours before the procedure, the availability of a significant dosage of the dye and the presence of abundant aqueous material (the bowel prep solution), taken together with peristaltic movements of the right colon, optimize the diffusion of the dye and contact of the dye with the different mucosa segments of the right colon.

Once the colonic mucosa is consistently and persistently coloured with methylene blue, the resulting diagnostic advantage is an increased ability to detect mucosal abnormalities according to different actions specifically related to the dye. First and foremost, areas of mucosa with inflammatory or neoplastic changes tend to decrease the uptake of the dye thus resulting in unstained areas which are easily distinguished (during the endoscopic procedures) from normal mucosa which exhibits a homogeneous staining pattern. Another advantage of the oral composition disclosed herein is to provide an improved detection of the pathological and/or not pathological lesions in the area to be investigated by the endoscopic diagnosis, such as the colon regions in all its anatomical segments (ascending, descending, rectosigmoid and transverse colon). For example, the right part of the colon can be the more accurately stained area.

The oral composition disclosed herein allows, thanks to a different uptake of the dye in the intercellular and intracellular spaces, a contrast enhancing efficacy of the dye in perceiving the deep mucosal tissue structure with the cripta and the gland ducts, thus improving the exact definition of the lesions and/or the borders of the lesions that the endoscopist has to identify and take out. An improved definition of the mucosal tissue structure and organization of the lesions is ensured, allowing for early detection of the lesions.

The better definition of the lesions provided by the oral composition and administration schedule disclosed herein facilitates increased specificity and sensitivity of the detection of the lesions, thus reducing the occurrence of false-negatives and false-positives and allowing pathological or malignant areas to be more correctly identified and detected. In other words, the specific oral solid composition disclosed herein and the administration schedule of the solid composition defined herein provide the improved contrast of the dye on the mucosa tissue structures.

In particular, the oral solid composition and administration schedule disclosed herein enable very early detection of colon dysplasias and colon carcinomas, particularly of those resulting from previous ulcerative colitis or Crohn's disease.

A further advantage of the oral solid composition and administration schedule disclosed herein is to provide a maximized local bioavailability of the dye and an optimized biological effect of the same.

In fact, it should be noted that the dye in accordance with the disclosure herein is allowed to be locally released with an homogeneous spreading exactly in the place subjected to the endoscopic diagnosis. For example, as disclosed herein, the dye is released in the colon, including also the right part of the colon.

Thanks to the specific oral solid composition and to the defined administration schedule disclosed herein, the dye orally administered is locally released and also completely absorbed in the intestinal tract, such as in the colon and further such as in the right part of the colon. In that way, that which is disclosed herein avoids any undesired early release or early absorption in anatomical tracts such as the stomach or small intestine not of interest in the endoscopic diagnosis.

The localized absorption of the dye on the intestinal mucosa allows the dye to penetrate in the cells wherein it is retained leading to an improved staining effect, increased contrast and better detection and the related diagnosis.

Improved absorption of the dye is of particular relevance when methylene blue is used as the dye for the endoscopic diagnosis. That follows because methylene blue is a "vital dye" able to be uptaken by the cells in a different way than by the extracellular space. Moreover, oral administration of the composition defined herein according to the administration schedule disclosed herein can lead to detection of a larger number of lesions in the smaller size category, thus improving the endoscopic diagnosis.

The solid composition disclosed herein, administered orally as disclosed herein, advantageously can further extensively stain the colonic mucosas, reducing colonoscopy subjectivity due to the endoscopist or operator involved in the endoscopic diagnosis, and consequently improving efficacy of the diagnostic evaluation itself.

The oral composition disclosed herein also can reduce the time involved in the endoscopic diagnosis by avoiding the dead times involved with spraying the dye and then washing it out from the mucosas to be examined.

The examples below also clarify the oral composition and administration schedule disclosed herein, without entailing any restrictions whatsoever with respect thereto.

### EXAMPLES

### Example 1: controlled-release coated tablet for endoscopy (colon)

| Description | UOM | Amt. per tablet |
|---|---|---|
| **Components** | | |
| Carmine indigo | mg | 50.0 |
| Lecithin | mg | 5.0 |
| Stearic acid | mg | 10.0 |
| Mannitol | mg | 100.0 |
| Lactose | mg | 50.0 |
| Hydroxyethyl cellulose | mg | 25.0 |
| Sodium starch glycolate | mg | 6.0 |
| Colloidal hydrated silica | mg | 3.0 |
| Magnesium stearate | mg | 2.0 |

| **Coating** | | |
|---|---|---|
| Methacrylic acid copolymer type A (Eudragit L) | mg | 6.0 |
| Methacrylic acid copolymer type B (Eudragit S) | mg | 6.0 |
| Triethyl citrate | mg | 1.2 |
| talc | mg | 5.8 |
| Titanium dioxide | mg | 3.0 |

The applied process provides for mixing the dye with the lecithin surfactant, stearic acid, mannitol and half of the required amount of magnesium stearate. After compacting the mixture, followed by granulation, then cellulose, sodium starch glycolate, colloidal silica and the remaining magnesium stearate are added and, after further mixing, the final compression is then carried out to obtain 250 mg tablets. The tablet is then coated with a mixture of methacrylic copolymers of type A and B, so as to extend the resistance to dissolution in vitro up to a pH ≥7, characteristic of the ileocecal and colon environment.

### Example 2 : controlled-release release coated tablet for endoscopy (colon)

| Description | UOM | Amt. per tablet |
|---|---|---|
| **Components** | | |
| Methylene blue | mg | 50.0 |
| Lecithin | mg | 5.0 |
| Stearic acid | mg | 10.0 |
| Mannitol | mg | 100.0 |
| Dibasic Sodium phosphate | mg | 25.0 |
| Hydroxyproply methylcellulose | mg | 35.0 |
| Sodium starch glycolate | mg | 6.0 |
| Colloidal hydrated silica | mg | 2.0 |
| Magnesium stearate | mg | 2.0 |

| **Coating** | | |
|---|---|---|
| Methacrylic acid copolymer type A (Eudragit L) | mg | 6.0 |
| Methacrylic acid copolymer type B (Eudragit S) | mg | 6.0 |
| Triethyl citrate | mg | 1.2 |
| talc | mg | 5.8 |
| Titanium dioxide | mg | 3.0 |

The preparation process provides for mixing the dye with lecithin, stearic acid and dibasic sodium phosphate, compaction thereof into wafers followed by dry granulation, mixing with the remaining components of the nucleus and the final compression to the weight of 235 mg/tablet. The coating uses methacrylic derivatives as base and an alcohol solvent to facilitate the application phase.

The tablets thus obtained were subjected to dissolution test in vitro, revealing a good resistance to the acid environment and a progressive transfer of the dye in the neutral environment having a pH at 7.2.

### Example 3: controlled release coated tablet for endoscopy (colon)

| Description | UOM | Amt. per tablet |
|---|---|---|
| **Components** | | |
| Methylene blue | mg | 200.0 |
| Lecithin | mg | 5.0 |
| Stearic acid | mg | 14.0 |
| Methylhydroxypropyl cellulose | mg | 180.0 |
| Mannitol | mg | 140.0 |
| Microcrystalline cellulose | mg | 140.0 |
| talc | mg | 10.0 |
| Colloidal hydrated silica | mg | 5.0 |
| Magnesium stearate | mg | 6.0 |

| **Coating** | | |
|---|---|---|
| Methacrylic acid copolymer type A (Eudragit L) | mg | 16.0 |
| Methacrylic acid copolymer type B (Eudragit S) | mg | 16.0 |
| Triethyl citrate | mg | 6,4 |
| talc | mg | 15.6 |
| Titanium dioxide | mg | 6.0 |

The composition is obtained through advance mixing and granulation of the dye, the lecithin as amphiphilic component, the stearic acid as a component of the lipophilic matrix, mannitol and part of the magnesium stearate. After screening the granules obtained preliminarily, the remaining components and in particular cellulose, capable of producing the hydrophilic matrix structure, are added. The final pharmaceutical form, obtained by compressing the mixture of powders and granules, and weighing about 720 mg, is subjected to coating with a mixture of copolymers of methacrylic derivatives of type A and B, supported by a plasticiser, i.e., triethyl citrate, by a dye pigment, i.e., titanium dioxide, and by an anti-stick agent, such as talc, using ethyl alcohol as a solvent.

The tablet thus obtained resists dissolution in vitro in buffers with pH < 2 and allows a progressive release of the dye substances in buffers with pH > 7 as here below detailed:
- Dissolution % after 2 hours in pH 1 dissolution medium: 0% (spec ≥10%)
- Dissolution % after 4 hour of pH 7.2 dissolution medium: 27%
- Dissolution % after 8 hour of pH 7.2 dissolution medium: 84% (spec >80%) The same tablets of this Example 3 have been used for a PK Phase I trial, where 200 and 400 mg single doses have been compared and where the following averaged values of the main PK parameters have been recorded: for the 200 mg dose
- mean t_{lag} ≥ 3 hours
- mean tₘₐₓ (hours) 16.10 ± 4.01
- bioavailability compared to injected dose (F_{abs} %): 139.19 ± 52.0
- mean Cₘₐₓ (ng/ml) 1662.2 ± 501.93
- urine excretion (mean% of the dose)= 39.67 ± 19.19
- mean t_{1/2} (hours) 20.19 ± 4.68,
whereas for the 400 mg dose the main parameters recorded have been:
- mean t_{lag} ≥3 hours
- mean tₘₐₓ (hours) 17.67 ± 3.60
- mean Cₘₐₓ (ng/ml) 1635.67 ± 729.57
- urine excretion (mean% of the dose)= 22.99 ± 14.92
- mean t_{1/2} (hours) 17.25 ± 7.43

### Example 4: controlled-release coated tablet for endoscopy (colon)

| Description | UOM | Amt. per tablet |
|---|---|---|
| **Tablet** | | |
| Indigo Carmine | mg | 100.0 |
| Sodium Lauryl sulphate | mg | 3.0 |
| Stearic acid | mg | 12.0 |
| Lactose | mg | 130.0 |
| Microcrystalline cellulose | mg | 80.0 |
| Sodium starch glycolate | mg | 10.0 |
| Colloidal hydrated silica | mg | 12.0 |
| Magnesium stearate | mg | 3.0 |

| **Coating** | | |
|---|---|---|
| Methacrylic acid copolymer type A | mg | 10.0 |
| Methacrylic acid copolymer type B | mg | 10.0 |
| Triethylcitrate | mg | 8.0 |
| Talc | mg | 6.0 |
| Titanium dioxide | mg | 3.8 |

The process provides for mixing the components of layer 1 and compression thereof, followed by the compression of a mixture of powders and granules obtained from a previous compaction of some components of the layer 2, precisely the dye, lecithin, stearic acid, the microcrystalline cellulose and mannitol with half of the magnesium stearate, with the remaining co-formulants.

The tablet, weighing about 250 mg, has two differently coloured distinct layers formulated for differentially releasing the dye both in the gastric sector and in the subsequent intestinal sector.

### Example 5: controlled-release coated tablet for endoscopy (colon)

| Description | UOM | Amt. per tablet |
|---|---|---|
| **Tablet** | | |
| Methylene blue | mg | 25.0 |
| Lecithin | mg | 3.0 |
| Stearic acid | mg | 10.0 |
| Methylhydroxypropyl cellulose | mg | 90.0 |
| Mannitol | mg | 121.0 |
| Microcrystalline cellulose | mg | 60.0 |
| talc | mg | 3.0 |
| Colloidal hydrated silica | mg | 5.0 |
| Magnesium stearate | mg | 3.0 |

| **Coating** | | |
|---|---|---|
| Methacrylic acid copolymer type A (Eudragit L) | mg | 8.0 |
| Methacrylic acid copolymer type B (Eudragit S) | mg | 8.0 |
| Triethyl citrate | mg | 3.2 |
| talc | mg | 7.8 |
| Titanium dioxide | mg | 3.0 |

The composition is obtained through ordered mixing of the dye, the lecithin as amphiphilic component, the stearic acid as component of the lipophilic matrix; then the remaining components were added and in particular the celluloses, capable of producing the hydrophilic matrix structure up to completion of the formula. The final pharmaceutical form, obtained by compressing the mixture of powders and granules, unitary weighing of about 320 mg, is subjected to coating with a mixture of copolymers of methacrylic derivatives of type A and B, supported by a plasticiser, triethyl citrate, by a dye pigment, titanium dioxide, and by an anti-sticking agent, such as talc, using ethyl alcohol or water or mixtures thereof as solvent.

The tablet thus obtained revealed in vitro a substantial non-dissolution (<10%) at pH 1 for 2 hours and a progressive dissolution in a simulated intestinal medium with pH 7.2 with a release of:
- about 10% after 1 hour (with specification limit ≤30%)
- about 44% after 4 hours and
- more than 90% at the eighth hour (with specification limit ≥80%).

The tablets have been used also to determine in Human volunteers, subjected to a standard bowel cleansing procedure through the administration of a 4-liters, PEG containing bowel preparation solution (commercially known as Selg^{®} Esse 1000), the PK characteristics of 2 doses of Methylene Blue administered as divided doses individually containing 25 mg of the dye.

The same tablets have been used for a PK Phase I trial, where 100 and 200 mg single doses have been compared and where the following averaged values of the main PK parameters have been recorded:
for the 100 mg dose
   - mean t_{lag} ≥3 hours
   - mean tₘₐₓ (hours) 12.0 (individual values 9- 16)
   - mean Cₘₐₓ (ng/ml) 573.60 ± 175.83
   - urine cumulative excretion (mean % of the dose) in 0 -60 hours = 28.02 ± 11.71
   - mean t_{l/2} (hours) 13.87 ± 5.09
whereas for the 200 mg dose the main parameters recorded have been
   - mean t_{lag} ≥3 hours
   - mean tₘₐₓ (hours) 16.0 (individual values 10- 24)
   - mean Cₘₐₓ (ng/ml) 1149.12 ± 261.95
   - urine cumulative excretion (mean % of the dose) in 0-60 hours = 38.67 ± 15.8
   - mean t_{1/2} (hours) 15.08 ± 5.85

In order to optimize the way to administer the tablets as function of the mucosal staining results, a clinical trial has been carried out with the above described tablets, using as discriminating parameter a scoring system (TSC) originally created and composed of a number between 0 and 20, calculated as sum of each individual staining score ranging 0 to 5 (where 0 is not stained at all, 1 is "traces", i.e. poor dye traces in colonic mucosa, 2 "detectable", i.e. relevant to a staining of at least 25% of the area, 3 is "acceptable", i.e. relevant to a staining of at least 50% of the area, 4 is "good", i.e. relevant to a staining of at least 75% of the area, 5 is "overstained", i.e. relevant to an overstaining not enabling an endoscopist to see the mucosal surface with the due accuracy in the 100% of the area), measured in the 4 segments of the colonic tract and indicated as right or ascending colon, transverse colon, descending colon and sigma-rectum; this scoring system was used to select the most reliable administration schedule of the dye with the aim of optimizing the tablets administration and the lesions detection possibilities during the colonoscopy procedure.

So, using the tablets formulated as described, the administration schedules has been changed on small groups of patients and the corresponding staining score has been determined. Since the importance of the colonic mucosal staining is that a well stained aspect should be extended to all the colonic segments, not only focused in a single colonic district, an additional parameter has been taken into account: the NSA or Number of Stained Area with staining score ≥2. With the application of these two parameters (TSC and NSA) the determination of the tablets administration schedule in order to obtain the best conditions for the endoscopist to enhance the detection of all the lesions in the colonic mucosa, has been carried out.

In the table below the different administration schedules of the two doses tested are reported with the corresponding measured staining score:
A) for 150 mg dose,
   - with the administration schedule A including 2 tablets (tbs.) before drinking the bowel prep, 2 tbs. after the first litre (L), 2 tbs. after the second Land the mean staining score was 6.8± 4.0 and the mean stained colonic segments (NSA) was 1.3.
   - with the administration schedule B including 6 tablets (tbs.) before drinking the bowel prep, the mean staining score was 2.3±2. 4 and the mean stained colonic segments (NSA) was 0,4.
   - with the administration schedule C including 6 tablets (tbs.) at the end of the bowel prep, the mean staining score was 8.1±3.6 and the mean stained colonic segments (NSA) was 1.5.
B) for 200 mg dose,
   - with the administration schedule D including 4 tablets (tbs.) before drinking the bowel prep, 2 tbs. after the first L, 2 tbs. after the second L and the mean staining score was 7.0± 5.0 and the mean stained colonic segments (NSA) was 1.3.
   - with the administration schedule E including 8 tablets (tbs.) at the end of bowel preparation solution the mean staining score was 9.8± 4.4 and the mean stained colonic segments (NSA) was 2.3 .
   - with the administration schedule F including 2 tablets (tbs.) before drinking the bowel prep, 2 tbs. after the first L, 2 tbs. after the second L and 2 tbs. at the end of bowel preparation the mean staining score was 9.3± 4.1 and the mean stained colonic segments (NSA) was 2.2.
   - with the administration schedule G including 2 tablets (tbs.) before drinking the bowel prep, 2 tbs. after the first L, 2 tbs. after the second L and 2 tbs. at the end of bowel preparation the mean staining score (TSC) was 10.5± 7.8 and the mean stained colonic segments (NSA) was 1.5.
   - with the administration schedule H including 4 tbs. after the third L, and 4 tbs. at the end of bowel preparation the mean staining score (TSC) was 10.0 ± 3.2 and the mean stained colonic segments (NSA) was 2.1.
   - with the administration schedule I including 4 tbs. after the second L and 4 tbs. after the third L of bowel preparation the mean staining score (TSC) was 11.4± 3.8 and the meanstained colonic segments (NSA) was 2.8.
   - with the administration schedule J including 2 tablets (tbs.) after the second L 3 tbs: after the third L and 3 tbs. at the end of bowel preparation the mean staining score (TSC) was 11.6± 3.5 and the stained colonic segments (NSA) was 2.6.

Using the same tablets described in Example 5, with a total dose of 200 mg of Methylene blue and an administration schedule of 2 tbs. after the second L, 3 after the third L and 3 at the end of bowel preparation, two Phase II clinical trials have been carried out:
A) on 96 completed patients for cancer screening and surveillance, and B) an additional 52 patients belonging to a high risk population, i.e. the patients with long standing Ulcerative Colitis.
   A) The cancer screening and surveillance trial had the aim of evaluating the polyp and adenoma detection rate in patients undergoing a full colonoscopy after colonic mucosal staining obtained with Methylene Blue MMX^{®} tablets. Therefore, the primary end-point was to evaluate the polyp detection rate and the adenoma detection rate after colonic mucosal staining

Other Secondary end-point(s) have been set, precisely:
- to classify polyps and adenomas detected after colonic mucosal staining
- to evaluate the serrated lesion detection rate.
- to evaluate the mucosal staining efficacy of Methylene Blue MMX^{®} tablets
- the Bowel cleansing quality was also evaluated according to the validated Boston Bowel Preparation Scale (BBPS).
- to collect data about safety and tolerability of Methylene Blue MMX^{®} tablets after administration of a single dose of 200 mg.

The subjects started the tablets intake in the afternoon before the colonoscopy day and had to drink at least 250 mL of preparation every 15 min, so that the bowel preparation intake could be completed 4 h after.

Measured trial variables:
- frequency of patients with polyps.
- Frequency of patients with adenomas.
- Number of adenomas in the right colon for each patient.
- Number of detected serrated lesions for each patient.
- Mucosal staining score for each area; total staining score.
- Boston bowel preparation score for bowel cleansing preparation quality.
- Time to reach the caecum.
- Time to withdrawal from caecum to exit;
- adverse events,
- vital signs (blood pressure, heart rate, saturation in peripheral blood), body weight.

The obtained results are here below summarized.

### 1) Mucosal abnormalities (polyps, adenomas and serrated lesions) in each colonic region per patient (A) and as total number (B)

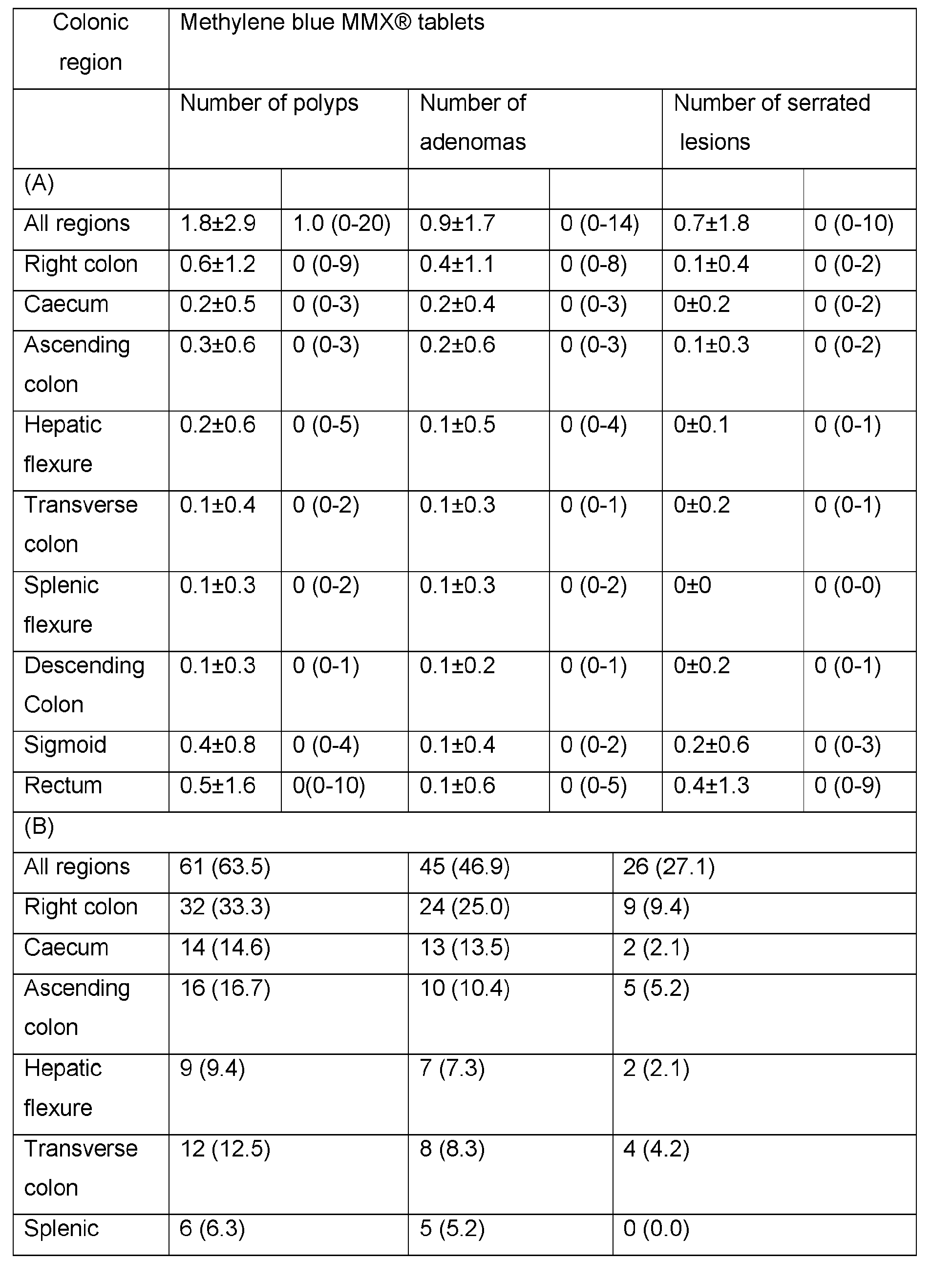

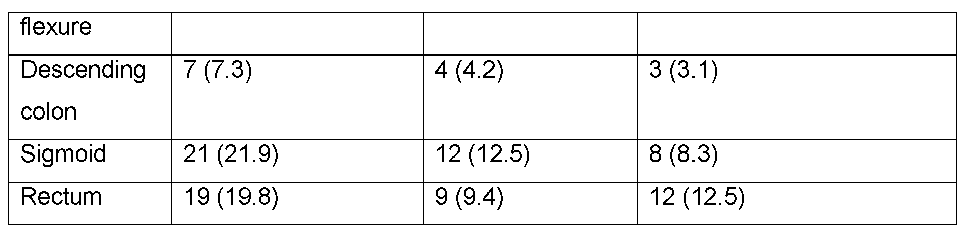

All endoscopic findings were classified by the histopathologist. The detected lesions were predominantly low grade tubular adenomas, hyperplastic serrated lesions, low grade serrated adenomas, low grade tubular-villous adenomas but also high grade adenomas with carcinoma in situ, including tubular-villous, villous and tubular lesions.

The mucosal staining efficacy of Methylene Blue MMX^{®} tablets was on average "acceptable" with the 50% of the mucosa stained in all 4 examined colonic regions. Bowel cleansing quality was on average "good" according to the total BBPS score.

### Conclusions:

The polyp detection rate and the adenoma detection rate/patient in the whole colon were on average 1.8±2.9 detected polyps and 0.9±1.7 detected adenomas. The polyp detection rate ranged from 0 to 20 polyps per subject and was higher in the rectum with a maximum of 10 polyps and in the right colon with a maximum of 9 lesions. The adenoma detection rate ranged from 0 to 14 adenomas per subject and was higher in the rectum with a maximum of 5 adenomas. In the right colon, the maximum detection rate was 8 detected adenomas. Serrated lesions ranged from 0 to 10, with the highest prevalence in the rectum with a maximum of 9 lesions.

As summarized in the following table. pPolyps were detected at a frequency of 64%, adenomas at a frequency of 47% and serrated lesions at a frequency of 27.1% (9% of subjects in the right colon, considered at the same severity level than adenomas).

| Number of patients with polyps (%) | Number of patients with adenomas (%) | Number of patients with serrated (%) |
|---|---|---|
| 61 (63.5) | 45 (46.9%) | 26 (27.1) |

There was a good consistency between the pit pattern scores and histological classification.

The most frequently affected region for polyps was sigmoid and rectum (21.9% and 19.8% respectively) and serrated lesions most frequently in the rectum (12.5%).Considering the 3 areas right, transverse and descending colon, the transverse colon is that with the lowest detection rate, followed by right and descending colon.

The analysis was performed also by subdividing the intraepithelial neoplasiae by size. The rate of detection by lesion size is summarised in the following table. The number of detected polyps, adenomas and serrated lesions < 5mm; mean (±SD) and median (range) are reported.

| Lesion Size | Methylene blue MMX^{®} tablets | | | | | |
|---|---|---|---|---|---|---|
| | Number of polyps | | Number of adenomas | | Number of serrated lesions | |
| ≤5mm | 1.3±2.3 | | 0.5±1.1 | | 0.6±1.7 | |

Smaller lesions (≤5 mm) were predominant in frequency, and that is remarkable inasmuch that the conventional white light colonoscopy, such smaller lesions are the most difficult to detect. Polyps: ≤5 mm had a maximum number of 15 detected abnormalities. The maximum number of detected adenomas ≤5 mm was 9 and 10 for the serrated lesions ≤5 mm.

Proportion of subjects with detected polyps by size, with detected adenomas and with detected serrated lesions presented also in the following summary table. The proportion of subjects with detected polyps, adenomas and serrated lesions by colonic region; number (%) of subjects is reported.

| | | Methylene blue MMX^{®} tablets | | |
|---|---|---|---|---|
| Population | Lesion Size | Subject with at least one polyp n(%) | Subjects with at least one adenoma n(%) | Subject with at least one serrated lesion n(%) |
| FAS (N=96) | ≤5 mm | 50 (52.1) | 30 (31.3) | 23 (24.0) |
| | 6-9 mm | 12 (12.5) | 10 (10.4) | 3 (3.1) |
| | ≥10 mm | 24 (25.0) | 22 (22.9) | 3 (3.1) |

Conclusions:
Efficacy of Methylene Blue MMX^{®} 25 mg modified release tablets was investigated and proved in the detection of the mucosal lesions in all the colonic districts, particularly with the lesions <5mm. A large proportion of patients, compared to data in the literature, has been found affected by the presence of polyps and adenomas, particularly in the sigmoid-rectum district and also in the right colon.
B) The efficacy of Methylene Blue MMX^{®} 25 mg modified release tablets was investigated in patients with ulcerative colitis with a diagnosis of ≥8 years and colitis activity index <8. This population was chosen because patients with long standing ulcerative colitis have a significantly higher risk for the development of colitis associated colorectal cancers.

The intraepithelial neoplasia detection rate was 16% (8 out of 50 subjects belonging to PP population) with a total of 10 intraepithelial neoplasiae detected in the 8 subjects.

Intraepithelial neoplasiae were most frequently found in the rectum-sigma segment (RES), followed by descending colon (DC) and transverse colon (TC) at the same frequency, and finally by the ascending colon (AC). The number of intraepithelial neoplasiae/subject was 0.2 ± 0.5.

As summarized below, false positive findings represented 8% (4 out of 50 subjects), whilst the false negative findings were 6% (3 out of 50). The method had a sensitivity greater than 50% (precisely 57.1 %) and a specificity greater that 90% (precisely 90.7%.)

Study results are consistent with the higher range of the literature data obtained with the chromo-endoscopy technology a spray of the dye instead of the oral administration of the dye during bowel preparation as disclosed herein. The dye spray technology was able to dramatically reduce the time of examination compared to the random biopsies:
in the cited spray chromo-endoscopy trial, intraepithelial neoplasiae were detected at a rate of 15.48% in the same population, with a solution of 0.1% methylene blue sprayed using a catheter.

Detection rate of intraepithelial neoplasiae and true and false positive and negative findings analysis population (N=52).

| Proportion of subjects with intraepithelial neoplasiae | True positive findings | False positive findings | True negative findings | False negative findings |
|---|---|---|---|---|
| 8 (15.4) | 4 (7.7) | 4(7.7) | 41 (78.8) | 3 (5.8) |

The mucosal staining efficacy of Methylene Blue MMX^{®} tablets was confirmed on average "acceptable" with 50% of the mucosa stained in all 4 examined colonic regions, with the best stained colonic segment resulting in the ascending colon, the region where it's more difficult to find the dysplastic lesions. The majority of subjects had NSA in all 4 regions. Bowel cleansing quality was on average "good" according to the total BBPS score.

Two images of colon endoscopy are below reported to also better clarify the invention. Image 1 shows the contrast enhancing efficacy of the dye according to the present invention in perceiving the deep mucosal tissue structure, with the foci of the glands well defined and darkened in a pre-polyp alteration of the colonic mucosa.

Image 2 shows the semi-continuous blue line defines exactly the borders of the colonic flat lesion that the endoscopist has to take out, allowing a better resolution of the lesion intervention and extraction. The tissue definition is absolutely enhanced owing to the orally administered dye as disclosed herein. With the conventional spraying techniques, the same performance cannot be obtained since little time is available between spray and observation (seconds or a couple of minutes).

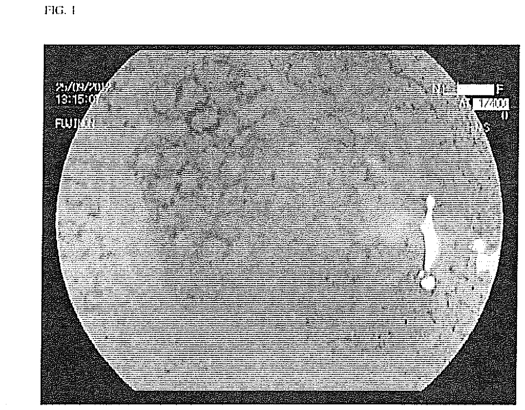

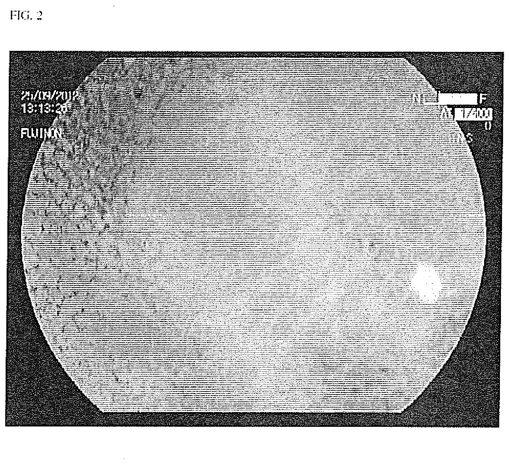

## Claims

1. Solid composition containing at least one dye in association with at least one physiologically acceptable excipient which comprises:
a) a lipophilic matrix which comprises lipophilic compounds with melting point below 90°C, and optionally an amphiphilic matrix, in which said at least one dye is at least partly incorporated,
b) an outer matrix which comprises hydrophilic compounds in which the lipophilic matrix, and optionally the amphiphilic matrix are dispersed;
c) optionally other physiologically acceptable excipients;
d) optional gastro-resistant coating
for use in endoscopic evaluation, **characterized in that** more than two unit dosages thereof are orally administered to a human according to a fractionated schedule in which a total amount from 100 to 400 mg, of said at least one dye is administered to said human in the 48 hours prior to the endoscopic evaluation wherein the total dose is fractionated in more than two unit dosages containing the same amount of dye, considered the active ingredient, and
wherein said at least one dye is selected from among methylene blue, congo red, indigo carmine, toluidine blue or mixtures thereof.

2. Solid composition for use as claimed in Claim 1, wherein the total dose is fractionated in four, six or eight dosage units.

3. The solid composition for use according to claim 1, wherein a total amount from 100 to 250 mg, more preferentially of 200 mg of said at least one dye is administered to said human.

4. The solid composition for use according to claim 1, wherein a total amount of 200 mg of said at least one dye is administered to said human

5. The solid composition for use according to claim 1, wherein a total amount of about 100 mg or about 200 mg of said at least one dye is administered to said human.

6. The solid composition for use according to claim 2, wherein said unit dosage contains individually from
20 mg to 100 mg by weight of said at least one dye or
25 mg or 50 mg by weight of said at least one dye or
about 200 mg by weight of said at least one dye.

7. The solid composition for use according to claim 1, wherein eight unit dosages thereof each containing about 25 mg by weight of said at least one dye are administered to said human in the 48 hours prior to the endoscopic evaluation or
wherein four unit dosages thereof each containing about 50 mg by weight of said at least one dye are administered to said human in the 48 hours prior to the endoscopic evaluation, or
wherein six unit dosages thereof each containing about 25 mg by weight of said at least one dye are administered to said human in the 48 hours prior to the endoscopic evaluation, or
wherein four unit dosages thereof each containing about 25 mg by weight of said at least one dye are administered to said human in the 48 hours prior to the endoscopic evaluation.

8. The solid composition for use according to claim 1, wherein the dosage units thereof are formulated as tablets, preferentially coated tablets, more preferentially gastro-protected coated tablets.

9. The solid composition for use according to claim 1, wherein the dosage units thereof each containing about 25 mg by weight of said at least one dye are administered to said human in the 24 or 48 hours prior to the endoscopic evaluation in a fractionated order at the beginning and/or during and/or at the end of a bowel cleansing preparation administration.

10. The solid composition for use according to claim 9, where the bowel cleansing preparation is consisting of a volume of 2 or more litres of a PEG-based salts containing solution or laxatives-based solution, or laxatives-based solution.

11. The solid composition for use according to claim 9, wherein eight unit dosages each containing about 25 mg by weight of said at least one dye in the 24-48 hours prior to the endoscopic evaluation are self administered to the patient by the patient himself, in a fractionated schedule at the beginning, during and at the end of the drinking of the bowel cleansing preparation.

12. The solid composition for use according to claim 9, wherein eight unit dosages each containing about 25 mg by weight of said at least one dye in the 24-48 hours prior to the endoscopic evaluation the unit dosages are self administered to the patient by the patient himself, in a fractionated schedule during administration of the bowel cleansing solution, preferentially taking a 2 tablets each 250 ml of solution during the drinking of the second, third and fourth liter of the bowel cleansing preparation, and more preferentially taking 5 tablets during the second and third liter and 3 tablets during and at the end of the fourth liter of bowel cleansing solution.

13. The solid composition for use according to claim 11, wherein the bowel cleansing preparation procedure is split in two steps of bowel preparation, preferentially to be administered in two days before the colonoscopy, preferentially part the day before the colonoscopy and part the same day of the colonoscopy and the unit dosages containing the dye are administered to the patient only during and/or at the end of the bowel preparation step carried out the day before the colonoscopy or
to be administered fractionated in both the day of the split bowel cleansing preparation, preferentially 6 during the bowel preparation of the day before the colonoscopy and 2 before and/or during and/or at the end of the drinking of bowel cleansing preparation the same day of the colonoscopy.

14. The solid composition for use according to claim 11, wherein eight unit dosages of the composition each containing about 25 mg by weight of said at least one dye are orally administered to a human according to a fractionated schedule in which a total amount of about 200 mg of said at least one dye is administered to said human in the 48 hours prior to the endoscopic evaluation in:
- 0 solid oral compositions after intake of the 1^{st} litre of cleansing solution;
- 3 solid oral compositions after intake of the 2^{nd} litre of cleansing solution
- 3 solid oral compositions after intake of the 3^{rd} litre of cleansing solution; and
- 2 solid oral compositions after intake of the 4^{th} (and last) litre of cleansing solution, or.
wherein eight unit dosages of the composition each containing about 25 mg by weight of said at least one dye are orally administered to a human according to a fractionated schedule in which a total amount of about 200 mg of said at least one dye is administered to said human in the 48 hours prior to the endoscopic evaluation in:
- 0 solid oral compositions after intake of the 1^{st} litre of cleansing solution;
- 4 solid oral compositions after intake of the 2^{nd} litre of cleansing solution
- 4 solid oral compositions after intake of the 3^{rd} litre of cleansing solution; and
- 0 solid oral compositions after intake of the 4^{th} (and last) litre of cleansing solution

15. The solid composition for use according to claim 9, wherein the cleansing solution is a saline and/or polyglycol solution, preferably an aqueous solution of polyethylene glycol.

16. The solid composition for use according to anyone of the preceding claims, wherein the endoscopic evaluation is endoscopic evaluation of inflammatory, ulcerative, pre-neoplastic, dysplastic and/or neoplastic pathologies and/or lesions of the gastrointestinal tract, preferably of the colon and/or
wherein the endoscopic evaluation is used to enhance the intestinal mucosal lesion detection the evaluation of cancerous forms, precancerous forms, interval cancers, adenomas, carcinomas, serrated lesions, intraepithelial neoplasias, dysplasias, polyps, pseudopolyps, pre-polyps or different inflammatory pathologies and/or lesions sessile, flat, peduncolated shape, and/or
wherein the endoscopic evaluation is used for the evaluation of right colon adenomas, right colon polyps and/or interval cancers and/or
wherein the endoscopic evaluation is used for the evaluation of small size lesions.

17. The solid composition for use of claim 16, wherein said small size lesions are lesions having a size equal to or less than 5 mm and/or wherein said small lesions are selected among polyps, adenomas and serrated lesions.

18. The solid composition for use of claim 1, wherein the endoscopic evaluation is for use for enhancing the detection of intestinal mucosal lesions for the early evaluation in a human previously suffering of other inflammatory pathology as, for example, Inflammatory Bowel Disease (IBD), Ulcerative Colitis or Crohn's Disease, and/or for use for enhancing the detection of intestinal mucosal lesions of the right part of the colon.

## Patentansprüche

1. Feststoffzusammensetzung, enthaltend mindestens einen Farbstoff in Verbindung mit mindestens einem physiologisch verträglichen Hilfsstoff, die Folgendes umfasst:
a) eine lipophile Matrix, die lipophile Verbindungen mit einem Schmelzpunkt unter 90 °C und optional eine amphiphile Matrix umfasst, in die der mindestens eine Farbstoff mindestens teilweise eingearbeitet ist,
b) eine äußere Matrix, die hydrophile Verbindungen umfasst, in der die lipophile Matrix und optional die amphiphile Matrix dispergiert sind;
c) optional weitere physiologisch verträgliche Hilfsstoffe;
d) optional einen magensaftresistenten Überzug
zur Verwendung bei einer endoskopischen Beurteilung, **dadurch gekennzeichnet, dass** mehr als zwei Einheitsdosierungen davon einem Menschen oral gemäß einem fraktionierten Schema verabreicht werden, in dem eine Gesamtmenge von 100 bis 400 mg des mindestens einen Farbstoffs dem Menschen in den 48 Stunden vor der endoskopischen Beurteilung verabreicht wird, wobei die Gesamtdosis in mehr als zwei Einheitsdosierungen fraktioniert wird, die die gleiche Menge an Farbstoff als Wirkstoff enthalten, und
wobei der mindestens eine Farbstoff ausgewählt ist aus Methylenblau, Kongorot, Indigokarmin, Toluidinblau oder Mischungen davon.

2. Feststoffzusammensetzung zur Verwendung nach Anspruch 1, wobei die Gesamtdosis in vier, sechs oder acht Dosierungseinheiten fraktioniert ist.

3. Feststoffzusammensetzung zur Verwendung nach Anspruch 1, wobei dem Menschen eine Gesamtmenge von 100 bis 250 mg, besonders bevorzugt von 200 mg des mindestens einen Farbstoffs verabreicht wird.

4. Feststoffzusammensetzung zur Verwendung nach Anspruch **1,** wobei dem Menschen eine Gesamtmenge von 200 mg des mindestens einen Farbstoffs verabreicht wird.

5. Feststoffzusammensetzung zur Verwendung nach Anspruch **1,** wobei dem Menschen eine Gesamtmenge von etwa 100 mg oder etwa 200 mg des mindestens einen Farbstoffs verabreicht wird.

6. Feststoffzusammensetzung zur Verwendung nach Anspruch 2, wobei die Einheitsdosierung einzeln einen Massenanteil von 20 mg bis 100 mg des mindestens einen Farbstoffs oder
einen Massenanteil von 25 mg oder 50 mg des mindestens einen Farbstoffs oder
einen Massenanteil von etwa 200 mg des mindestens einen Farbstoffs enthält.

7. Feststoffzusammensetzung zur Verwendung nach Anspruch 1, wobei dem Menschen in den 48 Stunden vor der endoskopischen Beurteilung acht Einheitsdosierungen davon verabreicht werden, die jeweils einen Massenanteil von etwa 25 mg des mindestens einen Farbstoffs enthalten, oder
wobei dem Menschen in den 48 Stunden vor der endoskopischen Beurteilung vier Einheitsdosierungen davon verabreicht werden, die jeweils einen Massenanteil von etwa 50 mg des mindestens einen Farbstoffs enthalten, oder
wobei dem Menschen in den 48 Stunden vor der endoskopischen Beurteilung sechs Einheitsdosierungen davon verabreicht werden, die jeweils einen Massenanteil von etwa 25 mg des mindestens einen Farbstoffs enthalten, oder
wobei dem Menschen in den 48 Stunden vor der endoskopischen Beurteilung vier Einheitsdosierungen davon verabreicht werden, die jeweils einen Massenanteil von etwa 25 mg des mindestens einen Farbstoffs enthalten.

8. Feststoffzusammensetzung zur Verwendung nach Anspruch 1, wobei die Dosierungseinheiten davon als Tabletten, bevorzugt überzogene Tabletten, besonders bevorzugt magensaftresistent überzogene Tabletten formuliert sind.

9. Feststoffzusammensetzung zur Verwendung nach Anspruch 1, wobei die Dosierungseinheiten davon, die jeweils einen Massenanteil von etwa 25 mg des mindestens einen Farbstoffs enthalten, dem Menschen in den 24 oder 48 Stunden vor der endoskopischen Beurteilung in fraktionierter Reihenfolge zu Beginn und/oder während und/oder am Ende einer Darmreinigungspräparatverabreichung verabreicht werden.

10. Feststoffzusammensetzung zur Verwendung nach Anspruch 9, wobei das Darmreinigungspräparat aus einem Volumen von 2 oder mehr Litern einer Salze auf PEG-Basis enthaltenden Lösung oder einer Lösung auf Laxativbasis oder aus einer Lösung auf Laxativbasis besteht.

11. Feststoffzusammensetzung zur Verwendung nach Anspruch 9, wobei dem Patienten in einem fraktionierten Schema zu Beginn, während und am Ende des Trinkens des Darmreinigungspräparats acht Einheitsdosierungen, die jeweils einen Massenanteil von etwa 25 mg des mindestens einen Farbstoffs enthalten, in den 24-48 Stunden vor der endoskopischen Beurteilung durch den Patienten selbst verabreicht werden.

12. Feststoffzusammensetzung zur Verwendung nach Anspruch 9, wobei dem Patienten in einem fraktionierten Schema während der Verabreichung der Darmreinigungslösung acht Einheitsdosierungen, die jeweils einen Massenanteil von etwa 25 mg des mindestens einen Farbstoffs enthalten, in den 24-48 Stunden vor der endoskopischen Beurteilung durch den Patienten selbst verabreicht werden, bevorzugt unter Einnahme von 2 Tabletten je 250 ml Lösung während des Trinkens des zweiten, dritten und vierten Liters des Darmreinigungspräparats, und besonders bevorzugt unter Einnahme von 5 Tabletten während des zweiten und dritten Liters und 3 Tabletten während und am Ende des vierten Liters der Darmreinigungslösung.

13. Feststoffzusammensetzung zur Verwendung nach Anspruch 11, wobei die Darmreinigungspräparatprozedur in zwei Schritte der Darmvorbereitung, bevorzugt zu verabreichen in zwei Tagen vor der Koloskopie, bevorzugt einen Teil an dem Tag vor der Koloskopie und einen Teil an dem gleichen Tag der Koloskopie, aufgeteilt wird und die Einheitsdosierungen, die den Farbstoff enthalten, dem Patienten nur während und/oder am Ende des Darmvorbereitungsschritts, der an dem Tag vor der Koloskopie durchgeführt wird, verabreicht werden, oder
zu verabreichen fraktioniert sowohl an dem Tag des aufgeteilten Darmreinigungspräparats, bevorzugt 6 während der Darmvorbereitung an dem Tag vor der Koloskopie und 2 vor und/oder während und/oder am Ende des Trinkens des Darmreinigungspräparats an dem selben Tag der Koloskopie.

14. Feststoffzusammensetzung zur Verwendung nach Anspruch 11, wobei acht Einheitsdosierungen der Zusammensetzung, die jeweils einen Massenanteil von etwa 25 mg des mindestens einen Farbstoffs enthalten, einem Menschen oral gemäß einem fraktionierten Schema verabreicht werden, in dem eine Gesamtmenge von etwa 200 mg des mindestens einen Farbstoffs dem Menschen in den 48 Stunden vor der endoskopischen Beurteilung verabreicht wird in:
0 oralen Feststoffzusammensetzungen nach Einnahme des 1. Liters Reinigungslösung;
3 oralen Feststoffzusammensetzungen nach Einnahme des 2. Liters Reinigungslösung
3 oralen Feststoffzusammensetzungen nach Einnahme des 3. Liters Reinigungslösung; und
2 oralen Feststoffzusammensetzungen nach Einnahme des 4. (und letzten) Liters Reinigungslösung, oder
wobei acht Einheitsdosierungen der Zusammensetzung, die jeweils einen Massenanteil von etwa 25 mg des mindestens einen Farbstoffs enthalten, einem Menschen oral gemäß einem fraktionierten Schema verabreicht werden, in dem eine Gesamtmenge von etwa 200 mg des mindestens einen Farbstoffs dem Menschen in den 48 Stunden vor der endoskopischen Beurteilung verabreicht wird in:
0 oralen Feststoffzusammensetzungen nach Einnahme des 1. Liters Reinigungslösung;
4 oralen Feststoffzusammensetzungen nach Einnahme des 2. Liters Reinigungslösung
4 oralen Feststoffzusammensetzungen nach Einnahme des 3. Liters Reinigungslösung; und
0 oralen Feststoffzusammensetzungen nach Einnahme des 4. (und letzten) Liters Reinigungslösung.

15. Feststoffzusammensetzung zur Verwendung nach Anspruch 9, wobei die Reinigungslösung eine Kochsalzlösung und/oder eine Polyglykollösung, bevorzugt eine wässrige Lösung von Polyethylenglykol, ist.

16. Feststoffzusammensetzung zur Verwendung nach einem der vorhergehenden Ansprüche, wobei die endoskopische Beurteilung eine endoskopische Beurteilung von entzündlichen, ulzerativen, präneoplastischen, dysplastischen und/oder neoplastischen Pathologien und/oder Läsionen des Gastrointestinaltrakts, bevorzugt des Dickdarms, ist, und/oder
wobei die endoskopische Beurteilung zur Verbesserung der Erkennung von Darmschleimhautläsionen zur Beurteilung von Krebsformen, Krebsvorstufen, Intervallkrebserkrankungen, Adenomen, Karzinomen, gezackten Läsionen, intraepithelialen Neoplasien, Dysplasien, Polypen, Pseudopolypen, Präpolypen oder verschiedenen entzündlichen Pathologien und/oder Läsionen mit sessiler, flacher, pedunkelartiger Form verwendet wird, und/oder
wobei die endoskopische Beurteilung zur Beurteilung von rechtsseitigen Dickdarmadenomen, rechtsseitigen Dickdarmpolypen und/oder Intervallkrebserkrankungen verwendet wird, und/oder
wobei die endoskopische Beurteilung zur Beurteilung von kleinformatigen Läsionen verwendet wird.

17. Feststoffzusammensetzung zur Verwendung nach Anspruch 16, wobei die kleinformatigen Läsionen Läsionen mit einer Größe gleich oder kleiner als 5 mm sind und/oder wobei die kleinformatigen Läsionen ausgewählt sind aus Polypen, Adenomen und gezackten Läsionen.

18. Feststoffzusammensetzung zur Verwendung nach Anspruch 1, wobei die endoskopische Beurteilung zur Verwendung zur Verbesserung der Erkennung von Darmschleimhautläsionen für die frühe Beurteilung bei einem Menschen dient, der bereits an einer anderen entzündlichen Pathologie wie beispielsweise einer chronisch-entzündlichen Darmerkrankung (IBD), Colitis ulcerosa oder Morbus Crohn leidet, und/oder zur Verwendung zur Verbesserung der Erkennung von Darmschleimhautläsionen des rechten Teils des Dickdarms dient.

## Revendications

1. Composition solide contenant au moins un colorant en association avec au moins un excipient physiologiquement acceptable qui comprend :
a) une matrice lipophile qui comprend des composés lipophiles ayant un point de fusion inférieur à 90 °C, et éventuellement une matrice amphiphile, dans laquelle ledit au moins un colorant est au moins en partie incorporé,
b) une matrice externe qui comprend des composés hydrophiles dans lesquels la matrice lipophile et éventuellement la matrice amphiphile sont dispersées ;
c) éventuellement d'autres excipients physiologiquement acceptables ;
d) un revêtement gastro-résistant facultatif
pour une utilisation dans une évaluation endoscopique, **caractérisée en ce que** plus de deux doses unitaires de celle-ci sont administrées par voie orale à un être humain selon un programme fractionné dans lequel une quantité totale de 100 à 100 mg dudit au moins un colorant est administrée audit être humain dans les 48 heures précédant l'évaluation endoscopique, dans laquelle la dose totale est fractionnée en plus de deux doses unitaires contenant la même quantité de colorant, considéré comme l'ingrédient actif, et
dans laquelle ledit au moins un colorant est choisi parmi le bleu de méthylène, le rouge Congo, le carmin d'indigo, le bleu de toluidine ou des mélanges de ceux-ci.

2. Composition solide pour une utilisation selon la revendication 1, dans laquelle la dose totale est fractionnée en quatre, six ou huit unités posologiques.

3. Composition solide pour une utilisation selon la revendication 1, dans laquelle une quantité totale de 100 à 250 mg, plus préférentiellement de 200 mg dudit au moins un colorant est administrée audit être humain.

4. Composition solide pour une utilisation selon la revendication 1, dans laquelle une quantité totale de 200 mg dudit au moins un colorant est administrée audit être humain.

5. Composition solide pour une utilisation selon la revendication 1, dans laquelle une quantité totale d'environ 100 à environ 200 mg dudit au moins un colorant est administrée audit être humain.

6. Composition solide pour une utilisation selon la revendication 2, dans laquelle ladite dose unitaire contient individuellement de 20 mg à 100 mg en poids dudit au moins un colorant ou
25 mg ou 50 mg en poids dudit au moins un colorant ou
environ 200 mg en poids dudit au moins un colorant.

7. Composition solide pour une utilisation selon la revendication 1, dans laquelle huit doses unitaires contenant chacune environ 25 mg en poids dudit au moins un colorant sont administrées audit être humain dans les 48 heures précédant l'évaluation endoscopique ou
dans laquelle quatre doses unitaires contenant chacune environ 50 mg en poids dudit au moins un colorant sont administrées audit être humain dans les 48 heures précédant l'évaluation endoscopique, ou
dans laquelle six doses unitaires contenant chacune environ 25 mg en poids dudit au moins un colorant sont administrées audit être humain dans les 48 heures précédant l'évaluation endoscopique, ou
dans laquelle quatre doses unitaires contenant chacune environ 25 mg en poids dudit au moins un colorant sont administrées audit être humain dans les 48 heures précédant l'évaluation endoscopique.

8. Composition solide pour une utilisation selon la revendication 1, dans laquelle les unités posologiques de celle-ci sont formulées sous forme de comprimés, de préférence de comprimés enrobés, plus préférentiellement de comprimés enrobés gastro-protégés.

9. Composition solide pour une utilisation selon la revendication 1, dans laquelle les unités posologiques contenant chacune environ 25 mg en poids dudit au moins un colorant sont administrées audit être humain dans les 24 ou 48 heures précédant l'évaluation endoscopique dans un ordre fractionné au début et/ou pendant et/ou à la fin d'une administration de préparation de nettoyage intestinal.

10. Composition solide pour une utilisation selon la revendication 9, dans laquelle la préparation de nettoyage intestinal est constituée d'un volume de 2 litres ou plus d'une solution contenant des sels à base de PEG ou d'une solution à base de laxatifs, ou d'une solution à base de laxatifs.

11. Composition solide pour une utilisation selon la revendication 9, dans laquelle huit doses unitaires contenant chacune environ 25 mg en poids dudit au moins un colorant dans les 24 à 48 heures précédant l'évaluation endoscopique sont auto-administrées au patient par le patient lui-même, selon un programme fractionné au début, pendant et à la fin de la consommation de la préparation de nettoyage intestinal.

12. Composition solide pour une utilisation selon la revendication 9, dans laquelle huit doses unitaires contenant chacune environ 25 mg en poids dudit au moins un colorant dans les 24 à 48 heures précédant l'évaluation endoscopique, les doses unitaires sont auto-administrées au patient par le patient lui-même, selon un programme fractionné pendant l'administration de la solution de nettoyage intestinal, en prenant de préférence 2 comprimés tous les 250 ml de solution pendant la consommation du deuxième, du troisième et du quatrième litre de la préparation de nettoyage intestinal, et plus préférentiellement en prenant 5 comprimés pendant le deuxième et le troisième litre et 3 comprimés pendant et à la fin du quatrième litre de solution de nettoyage intestinal.

13. Composition solide pour une utilisation selon la revendication 11, dans laquelle la procédure de préparation de nettoyage intestinal est divisée en deux étapes de préparation intestinale, à administrer de préférence deux jours avant la coloscopie, de préférence une partie la veille de la coloscopie et une partie le jour même de la coloscopie et les doses unitaires contenant le colorant sont administrées au patient uniquement pendant et/ou à la fin de l'étape de préparation intestinale effectuée la veille de la coloscopie ou
à administrer de manière fractionnée dans les deux jours de la préparation de lavage intestinal fractionnée, préférentiellement 6 pendant la préparation intestinale de la veille de la coloscopie et 2 avant et/ou pendant et/ou à la fin de la prise de préparation de lavage intestinal le jour même de la coloscopie.

14. Composition solide pour une utilisation selon la revendication 11, dans laquelle huit doses unitaires de la composition contenant chacune environ 25 mg en poids dudit au moins un colorant sont administrées par voie orale à un être humain selon un programme fractionné dans lequel une quantité totale d'environ 200 mg dudit au moins un colorant est administrée audit être humain dans les 48 heures précédant l'évaluation endoscopique dans :
- 0 composition orale solide après prise du 1^{er} litre de solution nettoyante ;
- 3 compositions orales solides après prise du 2^{ème} litre de solution nettoyante ;
- 3 compositions orales solides après prise du 3^{ème} litre de solution nettoyante ; et
- 2 compositions orales solides après la prise du 4^{ème} (et dernier) litre de solution nettoyante, ou
dans laquelle huit doses unitaires de la composition contenant chacune environ 25 mg en poids dudit au moins un colorant sont administrées par voie orale à un être humain selon un programme fractionné dans lequel une quantité totale d'environ 200 mg dudit au moins un colorant est administrée audit être humain dans les 48 heures précédant l'évaluation endoscopique dans :
- 0 composition orale solide après prise du 1^{er} litre de solution nettoyante ;
- 4 compositions orales solides après prise du 2^{ème} litre de solution nettoyante ;
- 4 compositions orales solides après prise du 3^{ème} litre de solution nettoyante ; et
- 0 composition orale solide après la prise du 4^{ème} (et dernier) litre de solution nettoyante.

15. Composition solide pour une utilisation selon la revendication 9, dans laquelle la solution nettoyante est une solution saline et/ou de polyglycol, de préférence une solution aqueuse de polyéthylène glycol.

16. Composition solide pour une utilisation selon l'une quelconque des revendications précédentes, dans laquelle l'évaluation endoscopique est une évaluation endoscopique de pathologies et/ou de lésions inflammatoires, ulcéreuses, pré-néoplasiques, dysplasiques et/ou néoplasiques du tractus gastro-intestinal, de préférence du côlon et/ou
dans laquelle l'évaluation endoscopique est utilisée pour améliorer la détection de lésions de la muqueuse intestinale, l'évaluation des formes cancéreuses, des formes précancéreuses, des cancers d'intervalle, des adénomes, des carcinomes, des lésions dentelées, des néoplasies intraépithéliales, des dysplasies, des polypes, des pseudopolypes, des prépolypes ou différentes pathologies inflammatoires et/ou des lésions de forme sessile, plate, pédonculée, et/ou
dans laquelle l'évaluation endoscopique est utilisée pour l'évaluation des adénomes du côlon droit, des polypes du côlon droit et/ou des cancers d'intervalle et/ou
dans laquelle l'évaluation endoscopique est utilisée pour l'évaluation des lésions de petite taille.

17. Composition solide pour une selon la revendication 16, dans laquelle lesdites lésions de petite taille sont des lésions ayant une taille égale ou inférieure à 5 mm et/ou dans laquelle lesdites petites lésions sont choisies parmi les polypes, les adénomes et les lésions dentelées.

18. Composition solide pour une utilisation selon la revendication 1, dans laquelle l'évaluation endoscopique est pour une utilisation pour améliorer la détection de lésions de la muqueuse intestinale pour l'évaluation précoce chez un être humain souffrant auparavant d'une autre pathologie inflammatoire telle que, par exemple, une maladie inflammatoire chronique de l'intestin (MICI), une rectocolite hémorragique ou la maladie de Crohn, et/ou pour améliorer la détection de lésions de la muqueuse intestinale de la partie droite du côlon.
